# EUROPÄISCHE PATENTSCHRIFT

(11) **EP 3 082 884 B1**
(45) Veröffentlichungstag und Bekanntmachung des Hinweises auf die Patenterteilung: **28.02.2018**
(21) Anmeldenummer: 14809012.9
(22) Anmeldetag: 08.12.2014
(51) Int. Cl.: A61L 9/01, C11D 3/20, C11D 3/50, A61L 2/18, A61L 2/23, C11D 3/00, C11D 3/28

(54) **LUFTPFLEGE- UND REINIGUNGSMITTEL ENTHALTEND CNGA2-ANTAGONISTEN**
AIR CARE AND CLEANING PRODUCTS CONTAINING CNGA2 ANTAGONISTS
PRODUITS DÉSODORISANTS D'INTÉRIEUR ET NETTOYANTS CONTENANT DES ANTAGONISTES DE CNGA2

(30) Priorität: 19.12.2013 DE 102013226600
(43) Veröffentlichungstag der Anmeldung: 26.10.2016
(73) Patentinhaber: Henkel AG & Co. KGaA, 40589 Düsseldorf (DE)
(72) Erfinder: HUCHEL, Ursula, 50733 Köln (DE); KRAUTWURST, Dietmar, 84048 Mainburg (DE); GEITHE, Christiane, 06632 Freyburg/Unstrut (DE)
(74) Vertreter: Viering, Jentschura & Partner mbB Patent- und Rechtsanwälte
(86) Internationale Anmeldenummer: PCT/EP2014/076881
(87) Internationale Veröffentlichungsnummer: WO 2015/091055

(56) Entgegenhaltungen:
- EP-A1- 2 100 589
- EP-A1- 2 133 102
- EP-A1- 2 272 491
- EP-A1- 2 524 959
- EP-A2- 0 826 764
- WO-A1-2005/004825
- T.-Y. CHEN ET AL: "Odorant Inhibition of the Olfactory Cyclic Nucleotide-gated Channel with a Native Molecular Assembly", THE JOURNAL OF GENERAL PHYSIOLOGY, Bd. 128, Nr. 3, 1. September 2006 (2006-09-01), Seiten 365-371, XP055172744, ISSN: 0022-1295, DOI: 10.1085/jgp.200609577

## Beschreibung

Die vorliegende Erfindung liegt auf dem Gebiet der geruchsreduzierenden Stoffe und Duftstoffe, wie sie zum Beispiel in Wasch- oder Reinigungsmitteln, kosmetischen Mitteln sowie Luftpflegemitteln zum Einsatz kommen. Die Erfindung betrifft Mittel, die spezielle Verbindungen, die als Antagonisten des humanen olfaktorischen Ionenkanals cyclic nucleotide gated channel alpha 2 (CNGA2; NCBI Reference Sequence: NP_005131.1) wirksam sind, enthalten. Ferner betrifft die vorliegende Erfindung ein Verfahren zur Verminderung oder Vermeidung von Schlechtgerüchen unter Verwendung der oben erwähnten Mittel.

Wasch- oder Reinigungsmittel, kosmetische Mittel, wie Deodorants, bzw. Luftpflegemittel (z.B. Raumlufterfrischer, Raumdeodorant, Raumspray usw.), enthalten zumeist Duftstoffe, die den Mitteln einen angenehmen Geruch verleihen und gleichzeitig Schlechtgerüche olfaktorisch überdecken sollen. Die Duftstoffe maskieren dabei zumeist auch den Geruch anderer Inhaltstoffe, so dass beim Verbraucher ein angenehmer Geruchseindruck entsteht.

Alternativ können Schlechtgerüche durch geeignete Materialien absorbiert werden.

Als unangenehm empfundene Gerüche im WC/Bad-Bereich werden u.a. durch Indolverbindungen, wie Skatol (3-Methylindol) erzeugt. Skatol kommt im menschlichen Kot (bis zu 0,1%) vor und ist eine sehr intensiv und unangenehm riechende Verbindung.

Bisher werden Skatol und andere als unangenehm riechend empfundene Stoffe wie oben beschrieben entweder olfaktorisch durch Luftpflegemittel/Wasch- oder Reinigungsmittel überdeckt oder mittels geeigneter Materialien absorbiert. Beide Methoden der Schlechtgeruchsbekämpfung sind aber meist nicht vollständig und können somit das unangenehme Geruchserlebnis nicht beseitigen, sondern, wenn überhaupt, nur abschwächen.

Aufgabe der vorliegenden Erfindung war daher die Bereitstellung von Mitteln, die geeignet sind durch Skatol verursachte Schlechtgerüche olfaktorisch möglichst vollständig zu unterdrücken.

Gelöst wurde diese Aufgabe durch Luftpflegemittel, Wasch- oder Reinigungsmittel, die mindestens eine Verbindung enthalten, die als Antagonist des humanen olfaktorischen Ionenkanals cyclic nucleotide gated channel alpha 2 (CNGA2; NCBI Referenzsequenz: NP_005131.1; Accession NP_005131 XP_372263; Version NP_005131.1 GI:42718011; 18. April 2013) wirksam ist.

Es konnte überraschend gefunden werden, dass Skatol neben spezifischen Geruchsrezeptoren auch direkt den CNGA2 Ionenkanal von allen Geruchsrezeptorzellen aktiviert und damit das unangenehme Geruchserlebnis auslöst. Der CNGA2 Ionenkanal ist ein durch cAMP aktivierter Calciumkanal, der bei der Signalweiterleitung mittels Geruchsrezeptoren eine wichtige Rolle spielt. Bei der Geruchswahrnehmung werden durch die Duftstoffe G-Protein gekoppelte Transmembranrezeptoren (GPCR) aktiviert, die wiederum intrazellulär das Enzym Adenylatcyclase (AC) aktivieren, welches dann den Botenstoff cAMP produziert. cAMP wiederum aktiviert weitere intrazelluläre Signalmoleküle, wie beispielsweise Ionenkanäle und bestimmte Kinasen, was schließlich in einem zellulären Signal resultiert.

Die Inhibition der Aktivierung des CNGA2 Ionenkanals durch Skatol ist demnach ein geeigneter Ansatz um die Schlechtgeruchwahrnehmung, die durch Skatol ausgelöst wird, durch geeignete Antagonisten des CNGA2 Kanals zu unterdrücken. Die Anwendung derartiger Antagonisten des CNGA2 Kanals in Luftpflegemitteln oder Wasch- oder Reinigungsmitteln führt bei deren Anwendung daher zu einer verbesserten Unterdrückung von durch Skatol verursachten Schlechtgerüchen. Die Verbindung, die als CNGA2 Antagonist wirksam ist, ist eine Verbindung der Formel (I) 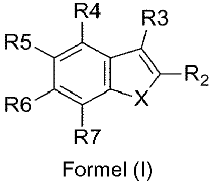 wobei
X für NR1 oder O steht;
R1 für eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen steht;
R2, R3, R4, R5, R6 und R7 jeweils unabhängig voneinander für Wasserstoff, ein Halogenatom, eine Aminogruppe, -NO₂, -NH-(C₁₋₄ Alkyl), -N(C₁₋₄ Alkyl)₂, eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen, -OH, oder eine -C(=O)-Y Gruppe stehen; und
Y für -OH, -O(C₁₋₄ Alkyl), -NH₂, NH-(C₁₋₄ Alkyl), oder -N(C₁₋₄ Alkyl)₂ steht.

In einer bevorzugten Ausführungsform der Erfindung ist die Verbindung, die als CNGA2 Antagonist wirksam ist, eine Verbindung der allgemeinen Formel (I), wobei
X für NR1 oder O steht;
R1 für eine Alkoxygruppe mit 1 bis 4 C-Atomen, eine Alkylgruppe mit 1 bis 4 C-Atomen, eine Alkenylgruppe mit 2 bis 4 C-Atomen, eine Alkinylgruppe mit 2 bis 4 C-Atomen steht;
R2, R3, R4, R5, R6 und R7 jeweils unabhängig voneinander für Wasserstoff, ein Halogenatom, eine Aminogruppe, -NO₂ -NH-(C₁₋₄ Alkyl), -N(C₁₋₄ Alkyl)₂, eine Alkoxygruppe mit 1 bis 4 C-Atomen, eine Alkylgruppe mit 1 bis 4 C-Atomen, eine Alkenylgruppe mit 2 bis 4 C-Atomen, eine Alkinylgruppe mit 2 bis 4 C-Atomen, -OH, oder eine -C(=O)-Y Gruppe stehen; und
Y für -OH, -O(C₁₋₄ Alkyl), -NH₂, NH-(C₁₋₄ Alkyl), oder -N(C₁₋₄ Alkyl)₂ steht.

Der Begriff "Antagonist", wie hierin im Zusammenhang mit dem CNGA2 Ionenkanal verwendet, bezeichnet Verbindungen, die an den CNGA2 Ionenkanal binden und dessen Aktivierung durch andere Bindungsmoleküle verhindern. Die hierin beschriebenen Antagonisten sind kompetitive Inhibitoren, die mit anderen Geruchsmolekülen, insbesondere Skatol, um die Bindungsstelle am CNGA2 Ionenkanal konkurrieren, diesen aber nicht oder zumindest nicht in dem gleichen Maße wie bekannte Aktivatoren, insbesondere Skatol, aktivieren. Die Affinität der Antagonisten ist vorzugsweise ausreichend hoch, damit sie wirksam mit Skatol um die Bindungsstelle am CNGA2 Kanal konkurrieren können. Im Rahmen einer bevorzugten Ausführungsform weist der CNGA2-Antagonist eine niedrigeren Kd-Wert und eine höhere Geruchsschwelle (jeweils als Konzentration in µM) auf, als ein CNGA2-Agonist. In verschiedenen Ausführungsformen haben daher geeignete Antagonisten einen K_{d} Wert von höchstens 100 µM, vorzugsweise höchstens 50 µM, noch bevorzugter höchstens 25 µM, besonders bevorzugt höchstens 10 µM, ganz besonders bevorzugt höchstens 0,1 µM, am bevorzugtesten höchstens 0,01 µM, aufweist. Der K_{d} Wert beschreibt dabei die Konzentration, bei der 50% der CNGA2 Ionenkanäle im Komplex mit dem Antagonisten vorliegen. Der K_{d} Wert kann mittels im Stand der Technik bekannter Verfahren, beispielsweise mittels Isothermer Titrationskalorimetrie (ITC), Oberflächenplasmonresonanz (SPR) oder Fluoreszenzspektroskopie, (jeweils bei einer Temperatur von 20 °C) bestimmt werden.

Ferner ist es (besonders gemeinsam mit den zuvor genannten bevorzugten Kd-Werten) bevorzugt, wenn der CNGA2-Antagonist eine Geruchsschwelle von mindestens 0,1 µM, bevorzugt von mindestens 10 µM, besonders bevorzugt von mindestens 25 µM, ganz besonders bevorzugt von mindestens 50 µM, am bevorzugtesten von mindestens 100 µM, aufweist.

In verschiedenen Ausführungsformen der Erfindung ist X = O und einer oder zwei, vorzugsweise einer von R2, R3, R4, R5, R6 und R7 C₁₋₄ Alkyl, insbesondere Methyl, und die übrigen sind

Wasserstoff. In verschiedenen anderen Ausführungsformen der Erfindung ist X = NR1, R1 ist C₁₋₄ Alkyl, insbesondere Methyl, und R2, R3, R4, R5, R6 und R7 sind Wasserstoff.

In verschiedenen Ausführungsformen wird die Verbindung, die als CNGA2 Antagonist wirksam ist, ausgewählt aus der Gruppe bestehend aus 1-Methylindol, 2-Methylbenzofuran, 3-Methylbenzofuran, 4-Methylbenzofuran, 5-Methylbenzofuran, 6-Methylbenzofuran und 7-Methylbenzofuran, insbesondere 3-Methylbenzofuran und 4-Methylbenzofuran.

In den hierin beschriebenen Luftpflegemitteln kann die Verbindung, die als CNGA2 Antagonist wirksam ist, in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,01 und 2 Gew.-%, jeweils bezogen auf das gesamte Mittel, enthalten sein.

Das Luftpflegemittel kann z.B. ein Raumlufterfrischer, Raumdeodorant oder Raumspray sein. In verschiedenen Ausführungsformen kann es zusätzlich einen oder mehrere Duftstoff(e) enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Ein weiterer Gegenstand der vorliegenden Erfindung ist ein Wasch- oder Reinigungsmittel enthaltend mindestens eine Verbindung, die als Antagonist des humanen olfaktorischen Ionenkanals CNGA2 wirksam ist, wobei die genannte Verbindung vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist. Bei geeigneten Reinigungsmitteln kann es sich z.B. um Reinigungsmittel für harte Oberflächen, wie vorzugsweise Haushaltsreiniger, Allzweckreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken. Bei geeigneten Waschmitteln kann es sich z.B. um ein Waschmittel im herkömmlichen Sinne handeln, oder um Waschhilfsmittel wie beispielsweise Vorbehandlungsmittel, Einweichmittel, Fleckensalz oder Nachbehandlungsmittel. Vorzugsweise handelt es sich um ein klassisches Waschmittel oder ein Nachbehandlungsmittel wie z.B. einen Weichspüler.

Die Verbindung, die als CNGA2 Antagonist wirksam ist und in den hierin beschriebenen Wasch- oder Reinigungsmitteln enthalten ist, ist in verschiedenen Ausführungsformen genauso definiert, wie oben im Zusammenhang mit den Luftpflegemitteln beschrieben.

Gemäß einer weiteren bevorzugten Ausführungsform liegt das erfindungsgemäße Mittel in fester oder flüssiger Form vor.

Nach einer weiteren bevorzugten Ausführungsform sind in einem erfindungsgemäßen Wasch- oder Reinigungsmittel ebenfalls zusätzliche Duftstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höher siedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester, Zimtsäurebenzylester.

Zu den leichter flüchtigen Duftstoffen zählen insbesondere die niedriger siedenden Riechstoffe natürlichen oder synthetischen Ursprungs, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Reinigungsmittel wenigstens eine, vorzugsweise mehrere, aktive Komponenten, insbesondere reinigungsaktive Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, antibakterielle Stoffe, Antioxidantien, Antistatika, Buildersubstanzen, Bleichmittel, Bleichaktivatoren, Bleichstabilisatoren, Bleichkatalysatoren, Cobuilder, Elektrolyte, Enzyme, Farbstoffe, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silikonöle, UV-Schutz-Substanzen, Viskositätsregulatoren und Verdickungsmittel. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen.

Gemäß einer bevorzugten Ausführungsform der Erfindung enthalten erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X-für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethylbenzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethylammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyl-oxyethyl)-N-(2-hydroxyethyl)ammonium-methosulfat. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart® bekannten Produkte der Firma BASF SE beziehungsweise die unter der Bezeichnung Rewoquat® bekannten Produkte des Herstellers EVONIK.

Tenside sind in erfindungsgemäßen Wasch- oder Reinigungsmitteln in Mengenanteilen von vorzugsweise 1 Gew.-% bis 50 Gew.-%, insbesondere von 2 Gew.-% bis 30 Gew.-%, enthalten.

Die Herstellung fester erfindungsgemäßer Mittel bereitet keine Schwierigkeiten und kann im Prinzip in bekannter Weise, zum Beispiel durch Sprühtrocknen oder Granulation, erfolgen. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Ein weiterer Gegenstand der Erfindung ist ein Verfahren zur Verminderung oder Vermeidung von Schlechtgerüchen, insbesondere im Sanitärbereich, d.h. im Bad/WC-Bereich, dadurch gekennzeichnet, dass ein Luftpflegemittel nach einem der Ansprüche 1 bis 5 auf eine Oberfläche

im Sanitärbereich aufgebracht wird oder die Oberflächen im Sanitärbereich mit einem Reinigungsmittel nach einem der Ansprüche 1 bis 6 gereinigt werden.

Bei den Oberflächen handelt es sich vorzugsweise um keramische Oberflächen im Bad/WC-Bereich, wie Waschbecken, Dusch- und Badewannen, WC-Becken und Urinale, insbesondere aber WC-Becken. Bei den beschriebenen Verfahren wird vorteilhafterweise ein Spülreiniger zum Einhängen in das WC-Becken verwendet.

### Beispiele

Für die durchgeführten Versuche wurde die Zelllinie HeLa/Olf (Shirokova et al., 2005, J. Biol. Chem. 280 (12): 11807-15; DE 103 50 054 A1) verwendet, die den orthologen Ionenkanal CNGA2 aus dem Rind (Bos taurus) stabil exprimiert. CNGA2 ist dabei nicht nur der Rezeptor für die getesteten Verbindungen, sondern auch der Effektor/Signalgeber, der als Ionenkanal nach Aktivierung ein extrazelluläres Ca²⁺-Signal in die Zellen hinein vermittelt, welches intrazellulär durch den Calcium-sensitiven Fluoreszenzfarbstoff FLUO-4 fluoreszenzspektroskopisch detektiert werden kann.

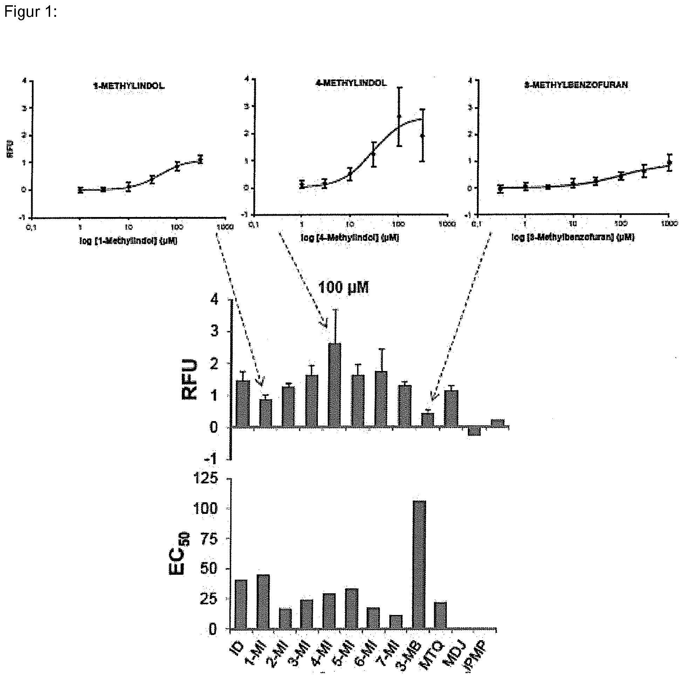

Die Zellen wurden in Gegenwart des Fluoreszenzfarbstoffes mit steigenden Konzentrationen der getesteten Verbindung kontaktiert und bei 20°C das resultierende Fluoreszenzsignal ausgelesen. Die Ergebnisse sind in Figur 1 dargestellt. Dabei zeigt Figur 1 exemplarisch die Titrationskurven von 1-Methylindol, 4-Methylindol und 3-Methylbenzofuran, das Balkendiagramm unter den Kurven zeigt die Amplitude des Fluoreszenzsignals bei einer Konzentration von 100 µM der jeweiligen Testverbindung (ID= Indol; 1-MI = 1-Methylindol; 2-MI = 2-Methylindol; 3-MI = 3-Methylindol; 4-MI = 4-Methylindol; 5-MI = 5-Methylindol; 6-MI = 6-Methylindol; 7-MI = 7-Methylindol; 3-MB = 3-Methylbenzofuran; MTQ = 6-Methyltetrahydrochinolin; MDJ = Methyldihydrojasmonat (Hedione); IPMP = 2-Isopropyl-3-Methoxypyrazin) und das Balkendiagramm darunter die EC₅₀ Werte, d.h. die Konzentration bei der eine halbmaximale Fluoreszenz zu beobachten war.

## Patentansprüche

1. Luftpflegemittel, enthaltend mindestens eine Verbindung, die als Antagonist des humanen olfaktorischen Ionenkanals cyclic nucleotide gated channel alpha 2 (CNGA2; NCBI Reference Sequence: NP_005131.1) wirksam ist, wobei
die Verbindung, die als CNGA2 Antagonist wirksam ist, eine Verbindung der Formel (I) ist 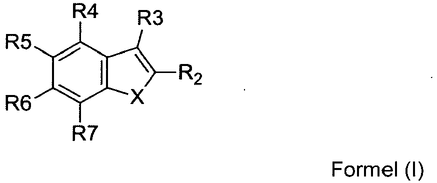 wobei
X für NR1 oder O steht;
R1 für eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen steht;
R2, R3, R4, R5, R6 und R7 jeweils unabhängig voneinander für Wasserstoff, ein Halogenatom, eine Aminogruppe, -NO₂ -NH-(C₁₋₄ Alkyl), -N(C₁₋₄ Alkyl)₂, eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen, -OH, oder eine -C(=O)-Y Gruppe stehen; und
Y für -OH, -O(C₁₋₄ Alkyl), -NH₂, NH-(C₁₋₄ Alkyl), oder -N(C₁₋₄ Alkyl)₂ steht,
wobei die Verbindung, die als CNGA2 Antagonist wirksam ist, in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,01 und 2 Gew.-%, jeweils bezogen auf das gesamte Mittel, enthalten ist und das Luftpflegemittel zusätzlich einen oder mehrere Duftstoff(e) enthält, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

2. Reinigungsmittel für harte Oberflächen, enthaltend mindestens eine Verbindung, die als Antagonist des humanen olfaktorischen Ionenkanals cyclic nucleotide gated channel alpha 2 (CNGA2; NCBI Reference Sequence: NP_005131.1) wirksam ist, wobei
die Verbindung, die als CNGA2 Antagonist wirksam ist, eine Verbindung der Formel (I) ist 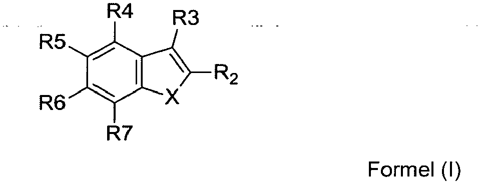 wobei
X für NR1 oder O steht;
R1 für eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen steht;
R2, R3, R4, R5, R6 und R7 jeweils unabhängig voneinander für Wasserstoff, ein Halogenatom, eine Aminogruppe, -NO₂ -NH-(C₁₋₄ Alkyl), -N(C₁₋₄ Alkyl)₂, eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen, -OH, oder eine -C(=O)-Y Gruppe stehen; und
Y für -OH, -O(C₁₋₄ Alkyl), -NH₂, NH-(C₁₋₄ Alkyl), oder -N(C₁₋₄ Alkyl)₂ steht,
wobei die genannte Verbindung vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist

3. Luftpflegemittel oder Reinigungsmittel nach einem der Ansprüche 1 oder 2, **dadurch gekennzeichnet, dass** die Verbindung, die als CNGA2 Antagonist wirksam ist, eine Verbindung der allgemeinen Formel (I), wobei
X für NR1 oder O steht;
R1 für eine Alkoxygruppe mit 1 bis 4 C-Atomen, eine Alkylgruppe mit 1 bis 4 C-Atomen, eine Alkenylgruppe mit 2 bis 4 C-Atomen, eine Alkinylgruppe mit 2 bis 4 C-Atomen steht;
R2, R3, R4, R5, R6 und R7 jeweils unabhängig voneinander für Wasserstoff, ein Halogenatom, eine Aminogruppe, -NO₂, -NH-(C₁₋₄ Alkyl), -N(C₁₋₄ Alkyl)₂, eine Alkoxygruppe mit 1 bis 4 C-Atomen, eine Alkylgruppe mit 1 bis 4 C-Atomen, eine Alkenylgruppe mit 2 bis 4 C-Atomen, eine Alkinylgruppe mit 2 bis 4 C-Atomen, -OH, oder eine -C(=O)-Y Gruppe stehen; und
Y für -OH, -O(C₁₋₄ Alkyl), -NH₂, NH-(C₁₋₄ Alkyl), oder -N(C₁₋₄ Alkyl)₂ steht.

4. Luftpflegemittel oder Reinigungsmittel nach einem der Ansprüche 1 bis 3, **dadurch gekennzeichnet, dass** die Verbindung, die als CNGA2 Antagonist wirksam ist, eine Verbindung der allgemeinen Formel (I), wobei
X = O und einer oder zwei, vorzugsweise einer von R2, R3, R4, R5, R6 und R7 C₁₋₄ Alkyl ist, insbesondere Methyl, und die übrigen Wasserstoff sind, oder wobei
X = NR1, wobei R1 C₁₋₄ Alkyl ist, insbesondere Methyl, und R2, R3, R4, R5, R6 und R7 Wasserstoff sind.

5. Luftpflegemittel oder Reinigungsmittel nach einem der Ansprüche 1 bis 4, **dadurch gekennzeichnet, dass** die Verbindung, die als CNGA2 Antagonist wirksam ist, ausgewählt wird aus der Gruppe bestehend aus 1-Methylindol, 2-Methylbenzofuran, 3-Methylbenzofuran, 4-Methylbenzofuran, 5-Methylbenzofuran, 6-Methylbenzofuran und 7-Methylbenzofuran.

6. Reinigungsmittel nach einem der Ansprüche 2 bis 5, **dadurch gekennzeichnet, dass**
(1) es mindestens ein Tensid ausgewählt aus der Gruppe bestehend aus anionischen, kationischen, nichtionischen, zwitterionischen, amphoteren Tensiden oder Mischungen daraus enthält; und/oder
(2) es in fester oder flüssiger Form vorliegt.

7. Verfahren zur Verminderung oder Vermeidung von Schlechtgerüchen, insbesondere im Sanitärbereich, **dadurch gekennzeichnet, dass** ein Luftpflegemittel nach einem der Ansprüche 1 und 3 bis 5 auf eine Oberfläche im Sanitärbereich aufgebracht wird oder die Oberflächen im Sanitärbereich mit einem Reinigungsmittel nach einem der Ansprüche 2 bis 6 gereinigt werden.

8. Verwendung eines Antagonisten des humanen olfaktorischen Ionenkanals cyclic nucleotide gated channel alpha 2 (CNGA2; NCBI Reference Sequence: NP_005131.1) in Luftpflege-, Wasch- oder Reinigungsmitteln zur Unterdrückung von durch Skatol verursachten Schlechtgerüchen, wobei der CNGA2 Antagonist eine Verbindung der Formel (I) ist 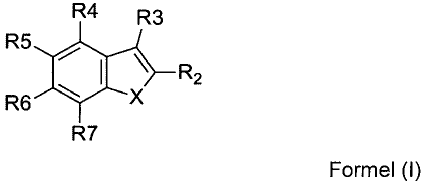 wobei ...
X für NR1 oder O steht;
R1 für eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen steht;
R2, R3, R4, R5, R6 und R7 jeweils unabhängig voneinander für Wasserstoff, ein Halogenatom, eine Aminogruppe, -NO₂ -NH-(C₁₋₄ Alkyl), -N(C₁₋₄ Alkyl)₂, eine lineare oder verzweigte, substituierte oder unsubstituierte Alkoxygruppe mit 1 bis 4 C-Atomen, eine lineare oder verzweigte, substituierte oder unsubstituierte Alkylgruppe mit 1 bis 4 C-Atomen, eine lineare oder verzweigte, substituierte oder unsubstituierte Alkenylgruppe mit 1 bis 4 C-Atomen, eine lineare oder verzweigte, substituierte oder unsubstituierte Alkinylgruppe mit 1 bis 4 C-Atomen, -OH, oder eine -C(=O)-Y Gruppe stehen; und
Y für Wasserstoff, C₁₋₄ Alkyl, -OH, -O(C₁₋₄ Alkyl), -NH₂, NH-(C₁₋₄ Alkyl), oder -N(C₁₋₄ Alkyl)₂ steht.

## Claims

1. An air care agent containing at least one compound that acts as an antagonist of the human olfactory ion channel cyclic nucleotide gated channel alpha 2 (CNGA2; NCBI reference sequence: NP_005131.1), wherein the compound that acts as a CNGA2 antagonist is a compound of formula (I) 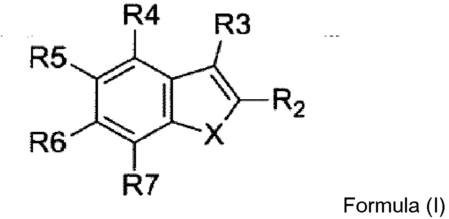 wherein
X represents NR1 or O;
R1 represents a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms;
R2, R3, R4, R5, R6 and R7 each represent, independently of one another, hydrogen, a halogen atom, an amino group, -NO₂ -NH-(C₁₋₄ alkyl), -N(C₁₋₄ alkyl)₂, a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms, -OH or a -C(=O)-Y group; and
Y represents -OH, -O(C₁₋₄ alkyl), -NH₂, NH-(C₁₋₄ alkyl) or -N(C₁₋₄ alkyl)₂,
wherein the compound that acts as a CNGA2 antagonist is contained in amounts of between 0.0001 and 50 wt.%, advantageously between 0.001 and 5 wt.%, more advantageously between 0.01 and 3 wt.%, in particular between 0.01 and 2 wt.%, based in each case on the entire agent, and the air care agent additionally contains one or more fragrance(s), in particular selected from the group comprising fragrances of natural or synthetic origin, preferably more volatile fragrances, fragrances having higher boiling points, solid fragrances and/or fragrances having good adhesion.

2. A cleaning agent for hard surfaces containing at least one compound that acts as an antagonist of the human olfactory ion channel cyclic nucleotide-gated channel alpha 2 (CNGA2; NCBI reference sequence: NP_005131.1), wherein the compound that acts as a CNGA2 antagonist is a compound of formula (I) 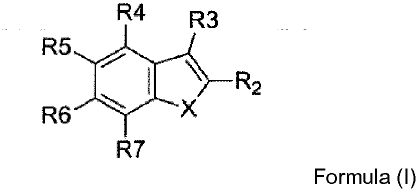 wherein
X represents NR1 or O;
R1 represents a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms;
R2, R3, R4, R5, R6 and R7 each represent, independently of one another, hydrogen, a halogen atom, an amino group, -NO₂ -NH-(C₁₋₄ alkyl), -N(C₁₋₄ alkyl)₂, a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms, -OH or a -C(=O)-Y group; and
Y represents -OH, -O(C₁₋₄ alkyl), -NH₂, NH-(C₁₋₄ alkyl) or-N(C₁₋₄alkyl)₂,
wherein said compound is preferably contained in amounts of between 0.0001 and 5 wt.%, advantageously between 0.001 and 4 wt.%, more advantageously between 0.01 and 3 wt.%, in particular between 0.1 and 2 wt.%, based in each case on the entire agent.

3. The air care agent or cleaning agent according to either claim 1 or claim 2, **characterized in that** the compound that acts as a CNGA2 antagonist is a compound of general formula (I), wherein
X represents NR1 or O;
R1 represents an alkoxy group having 1 to 4 C-atoms, an alkyl group having 1 to 4 C-atoms, an alkenyl group having 2 to 4 C-atoms, an alkinyl group having 2 to 4 C-atoms; R2, R3, R4, R5, R6 and R7 each represent, independently of one another, hydrogen, a halogen atom, an amino group, -NO₂, - NH-(C₁₋₄ alkyl), -N(C₁₋₄ alkyl)₂, an alkoxy group having 1 to 4 C-atoms, an alkyl group having 1 to 4 C-atoms, an alkenyl group having 2 to 4 C-atoms, an alkinyl group having 2 to 4 C-atoms, -OH or a - C(=O)-Y group; and Y represents -OH, -O(C₁₋₄alkyl), -NH₂, NH-(C₁₋₄ alkyl) or -N(C₁₋₄alkyl)₂.

4. The air care agent or cleaning agent according to one of claims 1 to 3, **characterized in that** the compound that acts as a CNGA2 antagonist is a compound of general formula (I), wherein
X = O, and one or two, preferably one, of R2, R3, R4, R5, R6 and R7 is C₁₋₄ alkyl, in particular methyl, and the others are hydrogen, or wherein
X = NR1, wherein R1 is C₁₋₄ alkyl, in particular methyl, and R2, R3, R4, R5, R6 and R7 are hydrogen.

5. The air care agent or cleaning agent according to one of claims 1 to 4, **characterized in that** the compound that acts as a CNGA2 antagonist is selected from the group consisting of 1-methylindole, 2-methylbenzofuran, 3-methylbenzofuran, 4-methylbenzofuran, 5-methylbenzofuran, 6-methylbenzofuran and 7-methylbenzofuran.

6. The cleaning agent according to one of claims 2 to 5, **characterized in that**
(1) said cleaning agent contains at least one surfactant selected from the group consisting of anionic, cationic, non-ionic, zwitterionic, amphoteric surfactants or mixtures thereof; and/or
(2) said cleaning agent is present in solid or liquid form.

7. A method for reducing or preventing unpleasant smells, in particular in the sanitary field, **characterized in that** an air care agent according to one of claims 1 and 3 to 5 is applied to a surface in the sanitary field, or the surfaces in the sanitary field are cleaned using a cleaning agent according to one of claims 2 to 6.

8. The use of an antagonist of the human olfactory ion channel cyclic nucleotide-gated channel alpha 2 (CNGA2; NCBI reference sequence: NP_005131.1) in air care agents, detergents or cleaning agents for suppressing unpleasant smells caused by scatole, wherein the CNGA2 antagonist is a compound of formula (I) 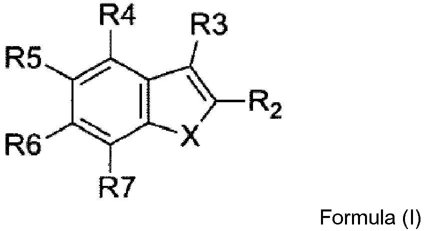 wherein
X represents NR1 or O;
R1 represents a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms;
R2, R3, R4, R5, R6 and R7 each represent, independently of one another, hydrogen, a halogen atom, an amino group, -NO₂ -NH-(C₁₋₄ alkyl), -N(C₁₋₄ alkyl)₂, a linear or branched, substituted or unsubstituted alkoxy group having 1 to 4 C-atoms, a linear or branched, substituted or unsubstituted alkyl group having 1 to 4 C-atoms, a linear or branched, substituted or unsubstituted alkenyl group having 1 to 4 C-atoms, a linear or branched, substituted or unsubstituted alkinyl group having 1 to 4 C-atoms, -OH or a -C(=O)-Y group; and
Y represents hydrogen, C₁₋₄ alkyl, -OH, -O(C₁₋₄ alkyl), -NH₂, NH-(C₁₋₄ alkyl) or -N(C₁₋₄ alkyl)₂.

## Revendications

1. Agent désodorisant d'intérieur, contenant au moins un composé agissant en tant qu'antagoniste du canal ionique olfactif humain « cyclic nucleotide gated channel alpha 2 » (CNGA2 ; séquence de référence NCBI : NP_005131.1), dans lequel le composé agissant en tant qu'antagoniste de CNGA2 est un composé de la formule (I) 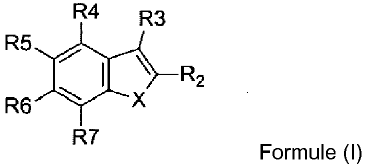 dans laquelle
X représente NR1 ou O;
R1 représente un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C ;
R2, R3, R4, R5, R6 et R7 représentent respectivement indépendamment l'un de l'autre de l'hydrogène, un atome d'halogène, un groupe amino, du -NO₂ du -NH-(C₁₋₄ alkyle), du -N(C₁₋₄ alkyle)₂, un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C, du -OH, ou un groupe -C(=O)-Y; et
Y représente du -OH, du -O(C₁₋₄ alkyle), du -NH₂, du NH-(C₁₋₄ alkyle), ou du -N(C₁₋₄ alkyle)₂,
dans lequel le composé agissant en tant qu'antagoniste de CNGA2 est présent dans des quantités comprises entre 0,0001 et 50 % en poids, de manière avantageuse entre 0,001 et 5 % en poids, de manière plus avantageuse entre 0,01 et 3 % en poids, notamment entre 0,01 et 2 % en poids, respectivement rapporté à l'ensemble de l'agent, et l'agent désodorisant d'intérieur contient de plus un ou plusieurs parfum(s), en particulier sélectionnés dans le groupe comprenant les parfums d'origine naturelle ou de synthèse, préférablement des parfums plus légèrement volatils, des parfums à point d'ébullition élevés, des parfums solides et/ou des parfums adhérents.

2. Agent nettoyant pour surfaces dures, contenant au moins un composé agissant en tant qu'antagoniste du canal ionique olfactif humain « cyclic nucleotide gated channel alpha 2 » (CNGA2 ; séquence de référence NCBI : NP_005131.1), dans lequel le composé agissant en tant qu'antagoniste de CNGA2 est un composé de la formule (I) 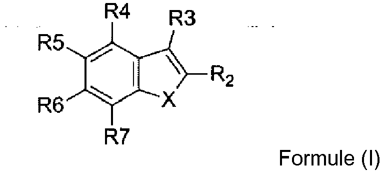 dans laquelle
X représente NR1 ou O ;
R1 représente un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C ;
R2, R3, R4, R5, R6 et R7 représentent respectivement indépendamment l'un de l'autre de l'hydrogène, un atome d'halogène, un groupe amino, du -NO₂ du -NH-(C₁₋₄ alkyle), du -N(C₁₋₄ alkyle)₂, un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C, du -OH, ou un groupe -C(=O)-Y; et
Y représente du -OH, du -O(C₁₋₄ alkyle), du -NH₂, du NH-(C₁₋₄ alkyle), ou du -N(C₁₋₄ alkyle)₂,
dans lequel le composé mentionné est présent dans des quantités comprises entre 0,0001 et 5 % en poids, de manière avantageuse entre 0,001 et 4 % en poids, de manière plus avantageuse entre 0,01 et 3 % en poids, notamment entre 0,1 et 2 % en poids, respectivement rapporté à l'ensemble de l'agent.

3. Agent désodorisant d'intérieur ou agent nettoyant selon l'une quelconque des revendications 1 ou 2, **caractérisé en ce que** le composé agissant en tant qu'antagoniste de CNGA2 est un composé de la formule générale (I), dans laquelle
X représente NR1 ou O ;
R1 représente un groupe alcoxyle comportant 1 à 4 atomes de C, un groupe alkyle comportant 1 à 4 atomes de C, un groupe alcényle comportant 2 à 4 atomes de C, un groupe alcynyle comportant 2 à 4 atomes de C ; R2, R3, R4, R5, R6 et R7 représentent respectivement indépendamment l'un de l'autre de l'hydrogène, un atome d'halogène, un groupe amino, du -NO₂ du -NH-(C₁₋₄ alkyle), du -N(C₁₋₄ alkyle)₂, un groupe alcoxyle comportant 1 à 4 atomes de C, un groupe alkyle comportant 1 à 4 atomes de C, un groupe alcényle comportant 2 à 4 atomes de C, un groupe alcynyle comportant 2 à 4 atomes de C, du -OH, ou un groupe -C(=O)-Y; et Y représente du -OH, du -O(C₁₋₄ alkyle), du -NH₂, du NH-(C₁₋₄ alkyle), ou du -N(C₁₋₄ alkyle)₂.

4. Agent désodorisant d'intérieur ou agent nettoyant selon l'une quelconque des revendications 1 à 3, **caractérisé en ce que** le composé agissant en tant qu'antagoniste de CNGA2 est un composé de la formule générale (I), dans laquelle
X = O et un ou deux, de préférence l'un de R2, R3, R4, R5, R6 et R7 est un alkyle en C1-4, en particulier du méthyle, et les autres sont de l'hydrogène, ou dans laquelle
X = NR1, dans laquelle R1 est un alkyle en C₁₋₄, en particulier du méthyle, et R2, R3, R4, R5, R6 et R7 sont de l'hydrogène.

5. Agent désodorisant d'intérieur ou agent nettoyant selon l'une quelconque des revendications 1 à 4, **caractérisé en ce que** le composé agissant en tant qu'antagoniste de CNGA2 est sélectionné dans le groupe constitué de 1-méthylindol, de 2-méthylbenzofurane, de 3-méthylbenzofurane, de 4-méthylbenzofurane, de 5-méthylbenzofurane, de 6-méthylbenzofurane et de 7-méthylbenzofurane.

6. Agent nettoyant selon l'une quelconque des revendications 2 à 5, **caractérisé en ce que**
(1) celui-ci contient au moins un tensioactif sélectionné dans le groupe constitué des tensioactifs anioniques, cationiques, non-ioniques, zwitterioniques, amphotères ou des mélanges de ceux-ci ; et/ou
(2) qu'il se présente sous forme solide ou liquide.

7. Procédé pour réduire ou éviter les mauvaises odeurs, en particulier dans le domaine sanitaire, **caractérisé en ce qu'**un agent désodorisant d'intérieur selon l'une quelconque des revendications 1 et 3 à 5 est appliqué sur une surface dans le domaine sanitaire ou que les surfaces du domaine sanitaire sont nettoyées avec un agent nettoyant selon l'une quelconque des revendications 2 à 6.

8. Utilisation d'un antagoniste du canal ionique olfactif humain « cyclic nucleotide gated channel alpha 2 » (CNGA2 ; séquence de référence NCBI : N_005131.1) dans des agents désodorisants d'intérieur, de lavage ou de nettoyage pour supprimer les mauvaises odeurs causées par le scatol, dans laquelle d'antagoniste de CNGA2 est un composé de la formule (I) 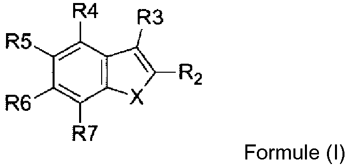 dans laquelle
X représente NR1 ou O ;
R1 représente un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C ;
R2, R3, R4, R5, R6 et R7 représentent respectivement indépendamment l'un de l'autre de l'hydrogène, un atome d'halogène, un groupe amino, du -NO₂ du -NH-(C₁₋₄ alkyle), du -N(C₁₋₄ alkyle)₂, un groupe alcoxyle linéaire ou ramifié, substitué ou non-substitué comportant 1 à 4 atomes de C, un groupe alkyle linéaire ou ramifié, substitué ou non-substitué comportant 1 à 4 atomes de C, un groupe alcényle linéaire ou ramifié, substitué ou non-substitué comportant 1 à 4 atomes de C, un groupe alcynyle linéaire ou ramifié, substitué ou non-substitué comportant 1 à 4 atomes de C, du -OH, ou un groupe -C(=O)-Y ; et
Y représente de l'hydrogène, du C₁₋₄ alkyle, du -OH, du -O(C₁₋₄ alkyle), du -NH₂, du NH-(C₁₋₄ alkyle), ou du -N(C₁₋₄ alkyle)₂.
